# EUROPÄISCHE PATENTSCHRIFT

(11) **EP 3 711 689 B1**
(45) Veröffentlichungstag und Bekanntmachung des Hinweises auf die Patenterteilung: **04.05.2022**
(21) Anmeldenummer: 20163226.2
(22) Anmeldetag: 16.03.2020
(51) Int. Cl.: A61B 17/29

(54) **SCHAFT FÜR EIN ENDOSKOPISCHES INSTRUMENT, ENDOSKOPISCHES INSTRUMENT SOWIE ENDOSKOPISCHES SYSTEM**
SHAFT FOR AN ENDOSCOPE INSTRUMENT, ENDOSCOPE INSTRUMENT AND ENDOSCOPE SYSTEM
TIGE POUR UN INSTRUMENT ENDOSCOPIQUE, INSTRUMENT ENDOSCOPIQUE AINSI QUE SYSTÈME ENDOSCOPIQUE

(30) Priorität: 20.03.2019 DE 102019107091
(43) Veröffentlichungstag der Anmeldung: 23.09.2020
(73) Patentinhaber: Karl Storz SE & Co. KG, 78532 Tuttlingen (DE)
(72) Erfinder: Kärcher, Daniel, 78532 Tuttlingen (DE); Stefan, Jochen, 78532 Tuttlingen (DE); Unger, Tobias, 78532 Tuttlingen (DE)

(56) Entgegenhaltungen:
- WO-A1-2012/061646
- US-A- 5 527 339
- US-A- 5 954 259
- US-A1- 2008 021 278

## Beschreibung

Die vorliegende Erfindung betrifft einen Schaft für ein endoskopisches Instrument, insbesondere für ein endoskopisches medizinisches Instrument. Ebenso betrifft die vorliegende Erfindung ein endoskopisches Instrument mit einem solchen Schaft sowie ein endoskopisches System.

Endoskopische Operationstechniken haben sich für eine Vielzahl chirurgischer Eingriffe durchgesetzt. Dabei wird durch eine natürliche oder eine mit Hilfe einer Inzision geschaffene künstliche Körperöffnung ein endoskopisches Instrumentarium, das insbesondere ein Endoskop und ein oder mehrere endoskopische Instrumente umfassen kann, zu einem im Körperinneren gelegenen Operationsgebiet geführt. Endoskopische Instrumente weisen hierfür einen lang erstreckten Schaft auf, an dessen distalem, d.h. benutzerfernen Ende ein Werkzeug zur Durchführung chirurgischer Manipulationen angeordnet ist, das von einem am proximalen, d.h. benutzernahen Ende des Schafts angeordneten Handgriff über ein im Schaft angeordnetes langerstrecktes Übertragungselement betätigt werden kann. Der Schaft kann starr oder flexibel ausgebildet sein. Der Handgriff verbleibt bei einem endoskopischen Eingriff außerhalb der Körperöffnung, während der Schaft mit dem Werkzeug durch die Körperöffnung eingeführt wird.

Endoskopische Instrumente weisen gemäß einer verbreiteten Bauart einen Arbeitseinsatz auf, der an seinem distalen Ende das Werkzeug trägt und der auch das Übertragungselement umfassen kann. Dabei ist der Arbeitseinsatz lösbar mit dem Schaft des Instruments verbunden. Insbesondere kann nach Durchführung eines endoskopischen Eingriffs das Instrument zur Reinigung und/oder Sterilisation zerlegt werden, wobei der Arbeitseinsatz von dem Schaft gelöst und aus einem Schaftrohr des Schafts entnommen wird; ferner kann der Handgriff vom Schaft getrennt werden. Ferner können je nach Art und Fortgang eines endoskopischen Eingriffs unterschiedliche Werkzeuge benötigt werden. Daher wird üblicherweise ein jeweils benötigter Arbeitseinsatz ausgewählt und in den Schaft eingesetzt und mit diesem verbunden. Ebenso kann ein Arbeitseinsatz mit einem ersten Werkzeugtyp, etwa einem Schneidwerkzeug, durch einen anderen Arbeitseinsatz mit einem anderen Werkzeugtyp, etwa einem Greifwerkzeug, ersetzt werden. Für die lösbare Verbindung des Arbeitseinsatzes mit dem distalen Endbereich des Schafts ist insbesondere eine Verbindung vorteilhaft, die nach Art einer Bajonettverbindung ausgestaltet ist.

In DE 100 38 576 C1 ist ein medizinisches Instrument mit einen Schaft und einem an einem distalen Ende des Schafts abnehmbar befestigten Werkzeug offenbart, wobei der Schaft einen Innenschaft und einen den Innenschaft umgebenden und relativ zu diesem längsverschiebbaren Rohrschaft aufweist. Das Werkzeug ist in einer relativ zum Innenschaft vorgeschobenen Verriegelungsstellung des Rohrschafts am Schaft verriegelt und in einer relativ zum Innenschaft zurückgezogenen Freigabestellung des Rohrschafts vom Schaft abnehmbar. Ein distales Ende des Innenschafts weist eine bajonettartige Ausnehmung mit einem sich zunächst axial und anschließend umfänglich erstreckenden Abschnitt auf, wobei am proximalen Ende des Werkzeugs ein Halter ausgebildet ist, der mit einem sich quererstreckenden Abschnitt in die bajonettartige Ausnehmung einsetzbar ist.

Gemäß DE 10 2012 007 648 A1 umfasst ein medizinisches Instrument einen Außenschaft, eine Handhabungseinrichtung am proximalen Ende des Außenschafts, ein Werkzeug am distalen Ende des Außenschafts, mit einer ersten Wirkeinrichtung für eine erste Funktion und einer zweiten Wirkeinrichtung für eine zweite Funktion, sowie eine erste und eine zweite Übertragungseinrichtung in dem Außenschaft zum Übertragen einer Kraft oder eines Drehmoments zum Steuern der ersten bzw. zweiten Wirkeinrichtung. Das Werkzeug und der Außenschaft weisen Kupplungseinrichtungen zum lösbaren mechanischen Verbinden des Werkzeugs mit dem distalen Ende des Außenschafts auf. Die Kupplungseinrichtungen können Bajonett-Kupplungseinrichtungen umfassen.

Gattungsgemäße Schäfte für medizinische endoskopische Instrumente werden von KARL STORZ SE & Co. KG beispielsweise unter der Bezeichnung "ClickLine^{®} Metall-Außenschaft" mit der Artikel-Nummer 33300 sowie unter der Bezeichnung "RoBi^{®} Metall-Außenschaft" mit der Artikel-Nummer 38600 vertrieben.

Durch die lösbare Verbindung zwischen dem Schaft und dem Arbeitseinsatz kann sich prinzipiell ein unerwünschtes Spiel einstellen, insbesondere aufgrund der vorgesehenen Toleranzfelder an der Verbindungsstelle zwischen Schaft und Arbeitseinsatz. Die Betätigung des Arbeitseinsatzes kann hierdurch beeinträchtigt werden, so kann beispielsweise ein sogenannter "toter Gang" zu einer ungenauen Betätigung des Werkzeugs führen. Bei der Realisierung engerer Toleranzfelder ergeben sich andererseits Einschränkungen hinsichtlich der Kompatibilität mit bereits vorhandenen Instrumenten, beispielsweise kann eine Verwendung eines einem breiteren Toleranzfeld entsprechenden Arbeitseinsatzes mit einem Schaft, der für engere Toleranzen ausgelegt ist, ausgeschlossen sein.

WO 2012/061646 A1 offenbart einen Schaft für ein endoskopisches Instrument, mit einem in einem distalen Endbereich des Schafts angeordneten Verbindungsabschnitt, wobei an dem Verbindungsabschnitt eine Verbindungseinrichtung zur Verbindung, insbesondere nach Art einer Bajonettverbindung, mit einem austauschbaren Arbeitseinsatz ausgebildet ist, wobei die Verbindungseinrichtung eine erste und zweite Eingriffsstruktur für einen Verbindungseingriff eines Eingriffselements eines Arbeitseinsatzes einer ersten Bauart aufweist.

Es ist Aufgabe der vorliegenden Erfindung, einen Schaft für ein endoskopisches Instrument, ein endoskopisches Instrument und ein endoskopisches System anzugeben, wobei die oben genannten Nachteile möglichst vermieden werden. Insbesondere ist es Aufgabe der Erfindung, einen Schaft für ein endoskopisches Instrument sowie ein endoskopisches Instrument und ein endoskopisches System anzugeben, wobei eine höhere Betätigungsgenauigkeit erreichbar ist und wobei gleichzeitig ein hohes Maß an Einsatzflexibilität und Kompatibilität gewährleistet ist.

Diese Aufgabe wird durch einen Schaft gemäß Anspruch 1, durch ein endoskopisches Instrument gemäß Anspruch 13 sowie durch ein endoskopisches System gemäß Anspruch 14 gelöst.

Vorteilhafte Weiterbildungen der Erfindung ergeben sich aus den Unteransprüchen.

Ein erfindungsgemäßer Schaft ist für ein endoskopisches Instrument, insbesondere für ein medizinisches endoskopisches Instrument, ausgebildet und kann somit zu minimalinvasiven chirurgischen Eingriffen eingesetzt werden. Ein solcher Schaft kann als Teil oder Baugruppe eines endoskopischen Instruments, insbesondere eines medizinischen endoskopischen Instruments, ausgebildet sein bzw. gemeinsam mit weiteren Komponenten zu einem endoskopischen Instrument zusammengesetzt werden. Der Schaft ist insbesondere starr oder im Wesentlichen starr ausgebildet und kann Teil oder Baugruppe eines starren endoskopischen Instruments sein. Andererseits kann der Schaft aber auch flexibel ausgebildet sein.

Ein erfindungsgemäßer Schaft weist einen Verbindungsabschnitt auf, der in einem distalen Endbereich des Schafts angeordnet ist. Der Verbindungsabschnitt kann beispielweise durch einen distalen Endabschnitt des Schafts oder eines Schaftrohrs, das von dem Schaft umfasst ist oder diesen darstellt, gebildet werden. An dem Verbindungsabschnitt ist eine Verbindungseinrichtung ausgebildet für eine lösbare Verbindung zwischen dem Schaft und einem Arbeitseinsatz, wodurch der Arbeitseinsatz austauschbar mit dem Schaft verbunden ist, d.h. ein Arbeitseinsatz kann durch einen anderen ausgetauscht werden. Ein solcher Arbeitseinsatz kann insbesondere ein chirurgisches Werkzeug aufweisen, das beispielsweise ein oder mehrere schwenkbar gelagerte Maulteile umfassen kann, welche durch eine Längsverschiebung eines mit den Maulteilen verbundenen, innerhalb des Schafts geführten Übertragungselements mit einem am proximalen Ende des Schafts angeordneten Handgriff betätigt werden können. Hierfür kann der Arbeitseinsatz zumindest abschnittsweise in den Schaft eingesetzt und mit dem Verbindungsabschnitt des Schafts verbunden werden. Auf diese Weise kann der Verbindungsabschnitt beispielsweise einen Abschnitt des Arbeitseinsatzes einfassen oder umgeben, so dass eine besonders sichere Verbindung zwischen dem Schaft und einem Arbeitseinsatz realisiert werden kann, wobei der Schaft zur Abstützung des Arbeitseinsatzes dient.

Die Verbindung ist insbesondere nach Art einer Bajonettverbindung ausgestaltet. Es ist demnach zwischen der Verbindungseinrichtung und einem Arbeitseinsatz insbesondere eine Verbindung nach Art eines Bajonettverschlusses erzeugbar. Eine derartige Bajonettverbindung kann auf einfache Weise, insbesondere mit nur geringem Handhabungsaufwand, erzeugt werden und gewährleistet gleichzeitig ein hohes Maß an Verbindungssicherheit. Zum Erzeugen einer Verbindung nach Art eines Bajonettverschlusses kann es vorgesehen sein, dass die beiden Verbindungspartner entlang ihrer Längsachsen ineinander gesteckt und durch entgegengesetztes Drehen gesichert bzw. fixiert werden können. Insbesondere kann ein Arbeitseinsatz in den Schaft hineingesteckt und anschließend relativ zum Schaft verdreht werden, also um eine Längsachse des Schafts gedreht werden. Dies ist mit nur wenigen Handgriffen durchführbar.

Die Verbindungseinrichtung weist eine Mehrzahl von Eingriffsstrukturen für den Verbindungseingriff von Arbeitseinsätzen unterschiedlicher Bauformen auf, welche hier als unterschiedliche Arbeitseinsatztypen bezeichnet werden. Hierfür umfasst die Verbindungseinrichtung mindestens eine erste Eingriffsstruktur für einen Verbindungseingriff eines Eingriffselements eines Arbeitseinsatzes einer ersten Bauart, d.h. eines ersten Arbeitseinsatztyps. Die der Verbindungseinrichtung zugeordnete Eingriffsstruktur kann insbesondere als Bajonett-Eingriffsstruktur und das dem Arbeitseinsatz zugeordnete Eingriffselement insbesondere als Bajonett-Eingriffselement oder Bajonett-Flügel bezeichnet werden. Durch einen bajonettartigen Eingriff des Eingriffselements des Arbeitseinsatzes der ersten Bauart in die erste Eingriffsstruktur der Verbindungseinrichtung kann insbesondere eine Bajonettverbindung hergestellt werden, durch die eine Basis des Arbeitseinsatzes in Längsrichtung des Schafts feststehend mit dessen distalem Endabschnitt verbunden wird, wobei durch eine entgegengesetzte Drehung um eine Längsachse des Schafts die Verbindung wieder gelöst werden kann.

Erfindungsgemäß umfasst die Verbindungseinrichtung weiterhin mindestens eine zweite Eingriffsstruktur für einen Verbindungseingriff eines Eingriffselements eines Arbeitseinsatzes einer zweiten Bauart, d.h. eines zweiten Arbeitseinsatztyps. Durch den Eingriff des Eingriffselements des Arbeitseinsatzes des zweiten Arbeitseinsatztyps in die zweite Eingriffsstruktur der Verbindungseinrichtung kann in entsprechender Weise eine Verbindung, insbesondere eine Bajonettverbindung geschaffen werden, durch welche eine Basis des Arbeitseinsatzes der zweiten Bauart in Längsrichtung des Schafts feststehend mit dessen distalem Endabschnitt verbunden wird, wobei die Verbindung ebenfalls durch eine entgegengesetzte Drehung um die Längsachse des Schafts gelöst werden kann.

Weiter erfindungsgemäß sind die erste und die zweite Eingriffsstruktur unterschiedlich angeordnet und/oder ausgebildet. Dies bedeutet, dass die erste und die zweite Eingriffsstruktur beispielsweise in unterschiedlichen Positionen, insbesondere separat voneinander, an dem Verbindungsabschnitt angeordnet sind und/oder unterschiedlich dimensioniert sind und/oder unterschiedliche Formen aufweisen.

Dadurch, dass die Verbindungseinrichtung eine Mehrzahl von unterschiedlichen Eingriffsstrukturen aufweist, wobei ein Arbeitseinsatztyp mit einer der Eingriffsstrukturen in Verbindungseingriff bringbar ist und wobei durch Austausch des Arbeitseinsatztyps durch einen anderen Arbeitseinsatztyp ein Verbindungseingriff mit einer anderen Eingriffsstruktur erzeugbar ist, wird es ermöglicht, Arbeitseinsätze unterschiedlicher Bauformen auf eine jeweils optimale Weise mit dem Verbindungsabschnitt und damit mit dem Schaft zu verbinden. Insbesondere können die unterschiedlichen Eingriffsstrukturen für unterschiedliche Verbindungsaufgaben bzw. Verbindungseigenschaften ausgelegt sein.

Es besteht durch die erfindungsgemäße Ausgestaltung beispielsweise die Möglichkeit, zumindest eine der Eingriffsstrukturen für bereits vorhandene Arbeitseinsatztypen und eine weitere Eingriffsstruktur für einen neuen Arbeitseinsatztyp auszulegen. Dabei können die unterschiedlichen Eingriffsstrukturen auch im Hinblick auf unterschiedliche Toleranzfelder der unterschiedlichen Arbeitseinsatztypen ausgebildet sein. Ebenso kann der Schaft aufgrund der Mehrzahl der Eingriffsstrukturen zusammen mit bereits vorhandenen Arbeitseinsatztypen verwendet werden. Dies gewährleistet ein hohes Maß an Austauschbarkeit und Vielseitigkeit der Verwendung des Schafts.

Gemäß der Erfindung sind die erste und die zweite Eingriffsstruktur derart unterschiedlich angeordnet und/oder ausgebildet, dass die erste Eingriffsstruktur den Verbindungseingriff mit dem Eingriffselement des Arbeitseinsatzes der ersten Bauart zulässt und einen Verbindungseingriff mit dem Eingriffselement des Arbeitseinsatzes der zweiten Bauart blockiert, und dass die zweite Eingriffsstruktur den Verbindungseingriff nur mit dem Eingriffselement des Arbeitseinsatzes der zweiten Bauart zulässt und einen Verbindungseingriff mit dem Eingriffselement des Arbeitseinsatzes der ersten Bauart blockiert. Hierdurch kann insbesondere ein Verbindungseingriff nur zwischen dem ersten Arbeitseinsatztyp und der ersten Eingriffsstruktur zugelassen und durch Austausch des Arbeitseinsatztyps ein Verbindungseingriff nur zwischen dem zweiten Arbeitseinsatztyp und der zweiten Eingriffsstruktur zugelassen werden, während Verbindungseingriffe zwischen dem Eingriffselement des ersten Arbeitseinsatztyps mit der zweiten Eingriffsstruktur und umgekehrt unmöglich sind. Eine derartige Ausgestaltung der Verbindungseinrichtung kann auch als eine mechanische Kodierung angesehen werden. Eine derartige mechanische Kodierung kann in vorteilhafter Weise durch unterschiedlich geformte, dimensionierte und/oder angeordnete Eingriffsstrukturen ausgebildet sein. Zur Erzeugung einer mechanischen Kodierung können die Eingriffsstrukturen demnach unterschiedliche Formen aufweisen, insbesondere jeweils eine eigene bzw. individuelle Formgebung aufweisen. Ebenso können die Eingriffsstrukturen zueinander unterschiedlich dimensioniert sein, so dass aufgrund der unterschiedlichen Größen der Eingriffsstruktur eine Kodierung gebildet wird. Schließlich besteht auch die Möglichkeit, durch spezifische Anordnung der Eingriffsstrukturen eine Kodierung zu bilden, insbesondere durch Anordnung der unterschiedlichen Eingriffsstrukturen an unterschiedlichen Abschnitten bzw. Positionen des Schafts.

Durch eine derartige Kodierung können die Betriebssicherheit sowie die Nutzerfreundlichkeit weiter verbessert werden. Durch eine solche Kodierung kann insbesondere die Herstellung einer korrekten Verbindung zwischen dem Schaft und einem Arbeitseinsatz erleichtert und die Gefahr von fehlerhaften oder unzureichenden Verbindungen zwischen Schaft und Arbeitseinsatz reduziert werden. Die Kodierung kann beispielsweise dazu ausgebildet sein, lediglich aufeinander abgestimmte Verbindungspaarungen zuzulassen und/oder ungewünschte Verbindungspaarungen zu blockieren.

In vorteilhafter Weise können die erste und die zweite Eingriffsstruktur dadurch unterschiedlich angeordnet sein, dass die zweite Eingriffsstruktur in einem im Vergleich mit der ersten Eingriffsstruktur unterschiedlichen Abstand von einem distalen Ende des Schafts angeordnet ist. Gemäß dieser Ausführungsform weist die Verbindungseinrichtung somit eine zweite Eingriffsstruktur für einen Verbindungseingriff eines Eingriffselements eines Arbeitseinsatzes der zweiten Bauart auf, wobei die zweite Eingriffsstruktur in einem Abstand von einem distalen Ende des Schafts angeordnet ist, der sich von einem entsprechenden Abstand der ersten Eingriffsstruktur unterscheidet, also kleiner oder größer ist, und zwar insbesondere um mehr als bei den jeweiligen Arbeitseinsatztypen ggf. vorhandenen Fertigungstoleranzen entspricht. Hierdurch kann auf einfache und sichere Weise ein Verbindungseingriff zwischen einem, in einem entsprechenden Abstand etwa von einem an das distale Ende ansetzbaren Anschlag angeordneten Eingriffselement eines Arbeitseinsatzes mit einer der jeweiligen Bauart des Arbeitseinsatzes zugeordneten Eingriffsstruktur gewährleistet und zugleich ein Eingriff mit einer anderen Bauart des Arbeitseinsatzes, bei der das Eingriffselement in einem hiervon verschiedenen Abstand angeordnet ist, verhindert werden.

Gemäß einer bevorzugten Ausführungsform der Erfindung weist die Verbindungseinrichtung wenigstens eine in Längsrichtung verlaufende Aussparung auf, insbesondere wenigstens einen Längsschlitz oder wenigstens eine Längsnut, wobei der Längsschlitz bzw. die Längsnut zur Längsführung eines Eingriffselements eines Arbeitseinsatzes gemäß der ersten und/oder der zweiten Bauart ausgebildet ist. Der Längsschlitz bzw. die Längsnut kann in Längsrichtung des Schafts verlaufen, insbesondere näherungsweise parallel zu einer Längsachse des Verbindungsabschnitts, und ist vorzugsweise in distaler Richtung offen, um ein Einführen des Eingriffselements beim Verbinden des Arbeitseinsatzes mit dem Schaft zu erleichtern. Durch den Längsschlitz bzw. die Längsnut kann beispielsweise ein radialer Vorsprung, der ein Eingriffselement eines Arbeitseinsatzes bildet, geführt werden. In besonders bevorzugter Weise ist der Längsschlitz bzw. die Längsnut zur Längsführung des Eingriffselements sowohl des ersten als auch des zweiten Arbeitseinsatztyps geeignet. Der Längsschlitz bzw. die Längsnut kann beispielsweise in einem distalen Endabschnitt eines Schaftrohrs ausgebildet sein, welcher distale Endabschnitt den Verbindungsabschnitt des Schafts darstellt, wobei die Längsnut vorzugsweise innenseitig im Schaftrohr ausgebildet ist.

Vorzugsweise werden die erste und die zweite Eingriffsstruktur jeweils durch eine in Querrichtung verlaufende Aussparung gebildet, insbesondere durch einen quer zur Längsachse des Schafts bzw. des Verbindungsabschnitts verlaufenden Querschlitz oder durch eine quer zur Längsachse verlaufende Quernut. Dabei ist der Querschlitz bzw. die Quernut der ersten Eingriffsstruktur zur Längsfixierung des Eingriffselements des Arbeitseinsatzes gemäß der ersten Bauart und der Querschlitz bzw. die Quernut der zweiten Eingriffsstruktur zur Längsfixierung des Eingriffselements des Arbeitseinsatzes der zweiten Bauart ausgebildet. Die Querschlitze bzw. Quernuten sind beispielsweise in einem distalen Endabschnitt eines Schaftrohrs ausgebildet, welcher den Verbindungsabschnitt des Schafts bildet, wobei die Quernut vorzugsweise innenseitig im Schaftrohr ausgebildet ist.

Weiter vorzugsweise sind die als Querschlitze bzw. Quernuten ausgebildeten Eingriffsstrukturen separat voneinander ausgebildet. In besonders bevorzugter Weise können die Eingriffsstrukturen bzw. die Querschlitze oder Quernuten jeweils quer zum Längsschlitz oder zur Längsnut verlaufen und in den Längsschlitz bzw. die Längsnut münden. Eine derartige Anordnung insbesondere von Längsschlitz und Querschlitzen kann fertigungstechnisch einfach realisiert werden. Zudem kann durch diese insbesondere die Möglichkeit gewährleistet werden, eine Verbindung nach Art eines Bajonettverschlusses mit einem Arbeitseinsatz herzustellen, ebenso wie durch eine entsprechende Anordnung von Längsnut und Quernuten oder auch von Längsschlitz und Quernuten sowie von Längsnut und Querschlitzen. Dabei kann durch einen Längsschlitz bzw. eine Längsnut ein Vorsprung oder ein korrespondierend ausgebildetes Formelement eines Arbeitseinsatzes in Längsrichtung des Schafts eingeführt und ausgehend von dem Längsschlitz bzw. der Längsnut in einen der Querschlitze bzw. Quernuten überführt werden. Die Querschlitze bzw. Quernuten dienen dabei zur Längsfixierung eines Vorsprungs oder eines korrespondierend zum jeweiligen Querschlitz bzw. der jeweiligen Quernut ausgebildeten Eingriffselements eines Arbeitseinsatzes. Dabei kann ein solcher Vorsprung oder ein solches Formelement eines Arbeitseinsatzes in radialer Richtung gegenüber benachbarten Abschnitten des Arbeitseinsatzes vorstehen.

Es kann weiterhin von Vorteil sein, wenn die Eingriffsstrukturen, insbesondere die als Querschlitze oder Quernuten ausgebildeten Eingriffsstrukturen, unabhängig voneinander und/oder in einer Längsrichtung des Verbindungsabschnitts beabstandet voneinander ausgebildet sind. In besonders bevorzugter Weise können die Querschlitze, insbesondere die einem Längsschlitz zugeordneten mindestens zwei Querschlitze bzw. die einer Längsnut zugeordneten mindestens zwei Quernuten, unterschiedliche Abstände zum distalen Ende des Schafts aufweisen. Hierdurch kann auf besonders einfache und handhabungssichere Weise eine insbesondere bajonettartig ausgestaltete Verbindung und zugleich eine mechanische Kodierung ermöglicht werden. So kann beispielsweise ein Querschlitz bzw. eine Quernut, der bzw. die weiter entfernt von dem distalen Ende des Schafts angeordnet ist, für die Herstellung einer Verbindung mit einem neuen Arbeitseinsatztyp ausgebildet sein, wobei durch den größeren Abstand vom distalen Ende sichergestellt werden kann, dass dieser Querschlitz bzw. diese Quernut von bisherigen Bauarten des Arbeitseinsatzes, bei denen das entsprechende Eingriffselement mit einem kürzeren Abstand vom distalen Ende angeordnet ist, für einen Verbindungseingriff nicht erreicht wird. Somit kann eine geeignete Zuordnung von Querschlitzen bzw. Quernuten zu bestimmten Arbeitseinsatztypen erfolgen.

In bevorzugter Weise sind die erste und die zweite Eingriffsstruktur dadurch unterschiedlich ausgebildet, dass die zweite Eingriffsstruktur zur ersten unterschiedlich dimensioniert ist. Gemäß dieser Ausführungsform weist die Verbindungseinrichtung somit eine zweite Eingriffsstruktur für einen Verbindungseingriff eines Eingriffselements eines Arbeitseinsatzes einer zweiten Bauart auf, wobei die zweite Eingriffsstruktur eine Dimensionierung aufweist, die sich von einer entsprechenden Dimensionierung der ersten Eingriffsstruktur unterscheidet. So kann etwa die zweite Eingriffsstruktur entsprechend einem Eingriffselement des zweiten Arbeitseinsatztyps in axialer und/oder radialer Richtung größer bzw. breiter oder tiefer ausgebildet sein als die dem ersten Arbeitseinsatztyp zugeordnete erste Eingriffsstruktur, und zwar insbesondere um mehr als ggf. vorhandenen Fertigungstoleranzen des jeweiligen Arbeitseinsatztyps entspricht. Hierdurch kann auf einfache und sichere Weise ein Verbindungseingriff zwischen einem entsprechend ausgestalteten Eingriffselement eines Arbeitseinsatzes mit einer für die jeweilige Bauart des Arbeitseinsatzes dimensionierten Eingriffsstruktur gewährleistet und zugleich ein Eingriff mit einer Bauart des Arbeitseinsatzes, bei der das Eingriffselement hiervon verschieden dimensioniert ist, verhindert werden.

Vorzugsweise sind die erste und die zweite Eingriffsstruktur dadurch unterschiedlich voneinander dimensioniert, dass sie unterschiedliche Breiten aufweisen, wobei als Breite insbesondere eine Dimensionierung in Längsrichtung des Verbindungsabschnitts bezeichnet wird. Insbesondere können die erste und die zweite Eingriffsstruktur als Querschlitze oder Quernuten ausgebildet sein, wobei der Querschlitz bzw. die Quernut der ersten Eingriffsstruktur eine von einer Breite des Querschlitzes bzw. der Quernut der zweiten Eingriffsstruktur unterschiedliche Breite hat. Beispielsweise können zwei Querschlitze, die einem Längsschlitz zugeordnet sind bzw. von demselben Längsschlitz ausgehen, eine unterschiedliche Breite aufweisen; Entsprechendes gilt für zwei Quernuten. Die unterschiedlichen Breiten der Querschlitze bzw. Quernuten können korrespondierend zu als Vorsprünge oder Formelemente ausgebildeten Eingriffselementen an unterschiedlichen Arbeitseinsatztypen ausgebildet sein. Ein Querschlitz bzw. eine Quernut mit verhältnismäßig geringer Breite kann somit den Verbindungseingriff eines zu breiten Vorsprungs oder Formelements eines anderen Arbeitseinsatztyps blockieren. So kann es etwa vorgesehen sein, dass ein Vorsprung oder Formelement eines ersten Arbeitseinsatztyps zwar durch den Längsschlitz bis zu unterschiedlichen Querschlitzen bzw. Quernuten geführt, aber nur in einen bestimmten Querschlitz bzw. eine bestimmte Quernut hineinbewegt werden kann. Hierdurch kann auf besonders einfache und sichere Weise erreicht werden, dass ein Verbindungseingriff zwischen einem entsprechend ausgestalteten Eingriffselement eines Arbeitseinsatzes der ersten Bauart nur mit der ersten Eingriffsstruktur und zwischen einem entsprechend ausgestalteten Eingriffselement des zweiten Arbeitseinsatztyps nur mit der zweiten Eingriffsstruktur ermöglicht wird.

Sofern die Eingriffsstrukturen als Quernuten ausgebildet sind, können die Quernuten der ersten und der zweiten Eingriffsstruktur alternativ oder zusätzlich zur unterschiedlichen Breite eine unterschiedliche Tiefe haben. Weiter alternativ oder zusätzlich können die Querschlitze bzw. Quernuten unterschiedliche Querschnittsformen aufweisen. Auch hierdurch kann auf einfache und sichere Weise eine entsprechende Kodierung realisiert werden.

Gemäß einer besonders bevorzugten Ausführungsform der Erfindung sind die Querschlitze bzw. Quernuten in der Weise unterschiedlich angeordnet und ausgebildet, dass ein Querschlitz bzw. eine Quernut mit größerem Abstand zu einem distalen Ende des Verbindungsabschnitts eine größere Breite aufweist als ein Querschlitz bzw. eine Quernut mit geringerem Abstand zu dem distalen Ende. Der Verbindungsabschnitt kann beispielsweise als distaler Endabschnitt eines Schaftrohrs ausgebildet sein, welcher zugleich den distalen Endabschnitt des Schafts bildet, so dass in diesem Fall der Querschlitz bzw. die Quernut mit der größeren Breite einen größerem Abstand vom distalen Ende des Schafts aufweist als der Querschlitz bzw. die Quernut mit geringerer Breite. Es kann auf diese Weise ein zweiter Arbeitseinsatztyp mit einem größer dimensionierten Vorsprung oder Formelement durch den Längsschlitz bzw. die Längsnut geführt werden, wobei aufgrund der größeren Dimensionierung ein Einführen in den Querschlitz bzw. die Quernut der ersten Eingriffsstruktur, der bzw. die eine geringere Breite hat, blockiert ist. Erst ein weiter vom distalen Ende des Verbindungsabschnitts bzw. des Schafts angeordneter Querschlitz bzw. eine entsprechende Quernut weist eine für den Verbindungseingriff mit dem Vorsprung oder Formelement des zweiten Arbeitseinsatztyps geeignete Breite auf. Demgegenüber kann der Querschlitz bzw. die Quernut mit geringerer Breite für einen ersten Arbeitseinsatztyp vorgesehen sein, dessen korrespondierender Vorsprung oder korrespondierendes Formelement entlang des Längsschlitzes bzw. der Längsnut nur bis zu dem Querschlitz bzw. der Quernut geringerer Breite geführt werden kann, beispielsweise aufgrund einer entsprechenden Anordnung des Vorsprungs bzw. Formelements, insbesondere aufgrund entsprechender Längenabmessungen des Arbeitseinsatzes der ersten Bauart. Durch eine derartige Anordnung und Dimensionierung von Querschlitzen kann in geeigneter Weise eine Zuordnung von Querschlitzen bzw. Quernuten zu verschiedenen Arbeitseinsatztypen erreicht werden. Es lassen sich hierdurch mit einem einzigen Schaft auf besonders vorteilhafte Weise unterschiedliche Verbindungsaufgaben realisieren.

Alternativ oder zusätzlich zu einer Anordnung der Eingriffsstrukturen, wobei diese in Längsrichtung des Schafts hintereinander oder mit unterschiedlichem Abstand zum distalen Ende des Schafts angeordnet sind, können die unterschiedlichen Eingriffsstrukturen auch relativ zu einer Längsachse des Schafts mit unterschiedlichem radialen Abstand angeordnet sein. In diesem Fall kann der Schaft, insbesondere im Bereich des Verbindungsabschnitts, aus zwei ineinander angeordneten Schaftabschnitten gebildet sein. Beispielsweise kann in einem ersten radialen Abstand zur Längsachse des Schafts ein erster Längsschlitz mit einem davon ausgehenden ersten Querschlitz vorgesehen sein, und in einem zweiten radialen Abstand zur Längsachse des Schafts, der beispielsweise größer ist als der erste radiale Abstand, kann ein zweiter Längsschlitz mit einem davon ausgehenden zweiten Querschlitz vorgesehen sein; alternativ können in entsprechender Weise Längsnuten und damit zusammenhängende Quernuten in unterschiedlichen radialen Abständen von der Längsachse vorgesehen sein. Auch hierdurch kann auf einfache und sichere Weise eine mechanische Kodierung realisierbar sein.

Gemäß einer bevorzugten Ausgestaltung können die unterschiedlichen Eingriffsstrukturen, insbesondere die als Querschlitze oder Quernuten ausgebildeten Eingriffsstrukturen, zur Erzeugung unterschiedlicher Toleranzfelder im Verbindungseingriff mit unterschiedlichen Arbeitseinsatztypen ausgebildet sein. So kann beispielsweise ein Querschlitz oder eine Quernut mit geringerem Abstand zum distalen Ende des Schafts für den Verbindungseingriff mit einem bereits vorhandenen Arbeitseinsatztyp ausgebildet sein und dementsprechend für ein verhältnismäßig breites Toleranzfeld konzipiert sein. Demgegenüber kann ein Querschlitz oder eine Quernut mit größerem Abstand zum distalen Ende des Schafts für den Verbindungseingriff mit einem neuen Arbeitseinsatztyp ausgebildet sein, bei welchem ein engeres Toleranzfeld realisiert werden kann. Dabei kann etwa bei dem neuen Arbeitseinsatztyp eine engere Paarungstoleranz genutzt werden, bezogen auf den Abstand zwischen dem distalen Ende des Verbindungsabschnitts und einer distalen Seite eines Querschlitzes, verglichen mit einem entsprechenden Abstand zwischen einer distalen Seite eines Vorsprungs, welcher beim Verbindungseingriff in den Querschlitz eingreift, und einem entsprechenden Anschlag des Arbeitseinsatzes. Zumindest eine der Eingriffsstrukturen kann somit hinsichtlich eines verhältnismäßig engen Toleranzfeldes ausgebildet sein, um bei einer entsprechenden Verbindung mit einem geeigneten Arbeitseinsatztyp das Spiel zwischen Schaft und Arbeitseinsatz zu reduzieren. Die Genauigkeit der Betätigung kann somit verbessert werden, da sich auf diese Weise ein etwaiger toter Gang verkleinern oder vollständig vermeiden lässt. Beispielsweise beim Einsatz von neuen Arbeitseinsatztypen kann daher ein geringeres Spiel zwischen Arbeitseinsatz und Schaft erzielt werden. Zudem kann durch eine solche Ausgestaltung vermieden werden, dass die jeweiligen Arbeitseinsatztypen in Verbindungseingriff mit einer ungeeigneten Eingriffsstruktur gelangen.

Vorzugsweise weist die Verbindungseinrichtung mehrere erste Eingriffsstrukturen und mehrere zweite Eingriffsstrukturen auf, wobei die mehreren ersten Eingriffsstrukturen in Umfangsrichtung des Verbindungsabschnitts zueinander versetzt angeordnet sind und die mehreren zweiten Eingriffsstrukturen ebenfalls in Umfangsrichtung zueinander versetzt angeordnet sind. Insbesondere kann die Verbindungseinrichtung zwei erste Eingriffsstrukturen und zwei zweite Eingriffsstrukturen aufweisen, die in Umfangsrichtung des Verbindungsabschnitts jeweils einander gegenüberliegend angeordnet sind. Beispielsweise kann die Verbindungseinrichtung zwei oder mehr Längsschlitze oder Längsnuten aufweisen, wodurch eine weiter verbesserte Führung beim Einführen eines zu verbindenden Arbeitseinsatzes gewährleistet und eine verbesserte Verbindung ermöglicht werden kann. Bevorzugt sind zwei Längsschlitze in Umfangsrichtung des Verbindungsabschnitts gegenüberliegend angeordnet, d.h. die Längsschlitze sind bezogen auf die Längsachse des Verbindungsabschnitts um 180° zueinander versetzt und somit symmetrisch angeordnet. Bei einer Verbindung mit einem Arbeitseinsatz kann auf diese Weise eine gleichmäßige Krafteinleitung sichergestellt werden. Weiter bevorzugt können je Längsschlitz wenigstens zwei Querschlitze vorgesehen sein. Bevorzugt können sämtliche Querschlitze von dem jeweiligen Längsschlitz ausgehend in Umfangsrichtung in derselben Orientierung verlaufen, etwa vom distalen Ende des Schafts aus gesehen im Uhrzeigersinn von dem jeweiligen Längsschlitz. In besonders bevorzugter Weise kann die Kombination aus einem Längsschlitz und wenigstens zwei Querschlitzen in identischer Weise mehrfach vorgesehen sein. Es können zwei oder mehr solcher Kombinationen relativ zu einer Längsachse des Schafts symmetrisch angeordnet oder ausgebildet sein. Im Übrigen gelten für jede Kombination aus einem Längsschlitz und wenigstens zwei Querschlitzen die voranstehenden Ausführungen in gleicher Weise, wobei anstelle von Schlitzen auch jeweils Nuten vorgesehen sein können.

Es kann weiterhin von Vorteil sein, wenn der Verbindungsabschnitt als Teil eines Außenschaftes ausgebildet ist, insbesondere als distaler Endabschnitt eines Schaftrohrs, das den Außenschaft bildet. Vorzugsweise ist das Schaftrohr, zumindest der Verbindungsabschnitt, starr ausgebildet. Ein derartiger Außenschaft kann bevorzugt mit einer Umhüllung versehen sein, durch die beispielsweise eine Schutzfunktion oder eine elektrische Isolierung bereitgestellt wird. So kann das Schaftrohr beispielsweise aus einem metallischen Material bestehen, etwa aus Edelstahl, und die Umhüllung aus einem Kunststoffmaterial, welches etwa in Form eines Schrumpfschlauchs auf der Außenseite des Schaftrohrs aufliegen kann. Der Außenschaft kann ferner zur Führung eines Übertragungselements im Inneren, beispielsweise einer Zugstange oder eines Innenschafts, ausgebildet sein. Durch ein solches Übertragungselement kann ein Werkzeug eines mit dem Schaft verbundenen Arbeitseinsatzes in vorteilhafter Weise betätigt werden. Der Außenschaft kann das Übertragungselement geeignet umgeben und beweglich führen. Der erfindungsgemäße Schaft kann insbesondere insgesamt als Außenschaft ausgebildet sein oder einen Außenschaft aufweisen, wobei der Verbindungsabschnitt in vorteilhafter Weise an einem distalen Ende des Außenschaftes ausgebildet ist.

Ein weiterer Aspekt der vorliegenden Erfindung betrifft ein endoskopisches Instrument, insbesondere ein medizinisches endoskopisches Instrument, mit einem Schaft, der wie zuvor beschrieben ausgebildet ist, und mit wenigstens einem Arbeitseinsatz, der durch die Verbindungseinrichtung des Schafts lösbar mit dem Verbindungsabschnitt des Schafts verbunden ist. Wird nun die Verbindung zwischen dem Arbeitseinsatz und dem Schaft gelöst, kann anstelle des entfernten Arbeitseinsatzes ein anderer Arbeitseinsatz, beispielsweise ein anderer Arbeitseinsatztyp, mit dem Schaft verbunden werden. Je nach Arbeitseinsatztyp kann dabei ein Verbindungseingriff mit unterschiedlichen Eingriffsstrukturen des Schafts hergestellt werden. Die Eingriffsstrukturen können demnach für unterschiedliche Verbindungsaufgaben ausgelegt oder konzipiert sein.

Der Arbeitseinsatz weist insbesondere ein chirurgisches Werkzeug auf, das durch ein oder mehrere schwenkbar gelagerte Maulteile gebildet werden kann, welche mit einem langerstreckten Übertragungselement verbunden sind und durch eine Längsverschiebung des Übertragungselements vom proximalen Ende des Schafts her betätigt werden können. Vorzugsweise umfasst der Arbeitseinsatz eine Basis, welche wie oben beschrieben zum Verbinden mit dem Verbindungsabschnitt insbesondere nach Art einer Bajonettverbindung ausgebildet ist und hierfür mindestens ein Eingriffselement trägt. Die Basis ist insbesondere abschnittsweise etwa zylindrisch ausgebildet und zur Einführung in den Verbindungsabschnitt des Schafts bemessen, wobei die Einführtiefe durch einen Anschlag begrenzt wird. Das mindestens eine Eingriffselement ist in einem solchen Abstand vom Anschlag angeordnet, dass es in Verbindungseingriff in eine Eingriffsstruktur des Verbindungsabschnitts gebracht werden kann. An einem distalen Ende der Basis ist beispielsweise eine Gabelvorrichtung angeordnet zur schwenkbaren Lagerung wenigstens eines Maulteils des chirurgischen Werkzeugs, welches beispielsweise zum Schneiden oder Fassen von Gewebe ausgebildet sein kann. Die Gabelvorrichtung kann zwei gegenüberliegende Schwenklager aufweisen. Ferner kann die Basis eine Durchführung für das mit den schwenkbaren Maulteilen verbundene Übertragungselement aufweisen und dementsprechend rohrförmig ausgebildet sein. Das Übertragungselement kann somit durch den Schaft hindurchgeführt sein und bis zur Gabelvorrichtung reichen. Am proximalen Ende des Schafts kann eine Handhabe, beispielsweise ein Handgriff, lösbar befestigt sein, welcher mit dem Übertragungselement verbunden werden kann, um eine Betätigung durch einen Nutzer auf das Übertragungselement und weiter auf das Werkzeug des Arbeitseinsatzes zu übertragen.

Ein weiterer Aspekt der vorliegenden Erfindung betrifft ein endoskopisches System, insbesondere ein medizinisches endoskopisches System, mit einem wie oben beschrieben ausgebildeten Schaft und mit mindestens einem Arbeitseinsatz der ersten Bauart und mindestens einem Arbeitseinsatz der zweiten Bauart. Dabei ist, wie oben beschrieben, der Arbeitseinsatz der ersten Bauart für den Verbindungseingriff mit der ersten Eingriffsstruktur des Verbindungsabschnitts des Schafts ausgebildet und der Arbeitseinsatz der zweiten Bauart für den Verbindungseingriff mit der zweiten Eingriffsstruktur des Verbindungsabschnitts. Vorzugsweise sind der Arbeitseinsatz der ersten und der der zweiten Bauart derart unterschiedlich ausgebildet, dass der Arbeitseinsatz der ersten Bauart nur einen Verbindungseingriff mit der ersten Eingriffsstruktur und der der zweiten Bauart nur einen Verbindungseingriff mit der zweiten Eingriffsstruktur ermöglicht. Insbesondere weist der Arbeitseinsatz der zweiten Bauart mindestens ein Eingriffselement auf, das im Vergleich zu mindestens einem Eingriffselement des Arbeitselements der ersten Bauart unterschiedlich angeordnet und/oder dimensioniert und/oder geformt ist und dadurch den Verbindungsabschnitt nur mit der jeweils zugeordneten Eingriffsstruktur zulässt. Das endoskopische System umfasst somit den Schaft und eine Mehrzahl an unterschiedlichen Arbeitseinsatztypen, die durch die Verbindungseinrichtung einzeln und zueinander austauschbar mit dem Verbindungsabschnitt des Schafts verbindbar sind. Das endoskopische System wird daher durch eine Zusammenstellung des Schafts mit mindestens einem Arbeitselement des erstens Arbeitseinsatztyps und mindestens einem Arbeitselement des zweiten Arbeitseinsatztyps gebildet, wobei, wie voranstehend in Bezug auf den Schaft beschrieben, je nach Arbeitseinsatztyp ein Verbindungseingriff mit den unterschiedlichen Eingriffsstrukturen des Schafts hergestellt werden kann.

Dadurch, dass ein erster Arbeitseinsatztyp mit der ersten Eingriffsstruktur in Verbindungseingriff bringbar ist und durch Austausch durch einen zweiten Arbeitseinsatztyp ein Verbindungseingriff mit der zweiten Eingriffsstruktur erzeugbar ist, können die Eingriffsstrukturen für unterschiedliche Verbindungsaufgaben ausgelegt sein und die Arbeitseinsätze entsprechend unterschiedlich ausgestaltet sein. Hierdurch können einerseits die Betriebssicherheit erhöht und gleichzeitig vielfältige Einsatzmöglichkeiten gewährleistet werden.

Gemäß einer besonders bevorzugten Ausgestaltung des endoskopischen Systems kann sich ein Toleranzfeld zwischen dem ersten Arbeitseinsatztyp und der korrespondierenden ersten Eingriffsstruktur unterscheiden von einem Toleranzfeld zwischen dem zweiten Arbeitseinsatztyp und der dazu korrespondierenden zweiten Eingriffsstruktur. Insbesondere können sich die jeweiligen Paarungstoleranzen unterscheiden. Dabei können beispielsweise für bestehende Arbeitseinsatztypen herkömmliche Toleranzfelder umgesetzt werden, so dass eine Rückwärtskompatibilität mit derartigen Arbeitseinsatztypen erhalten bleibt, und gleichzeitig kann für neue Arbeitseinsatztypen ein engeres Toleranzfeld ausgewählt und umgesetzt werden, so dass im Einsatz derartiger Arbeitseinsatztypen ein geringeres Maß an Spiel erreicht wird bzw. ein etwaiger toter Gang verkleinert oder ganz vermieden werden kann.

Gemäß einem erfindungsgemäßen Verfahren zum Konfigurieren eines endoskopischen Instruments wird ein endoskopisches Instrument bereitgestellt, das einen Schaft umfasst, der wie oben beschrieben ausgebildet ist, und einen Arbeitseinsatz gemäß der ersten Bauart, der mit dem Schaft verbunden ist. Dabei steht somit das mindestens eine Eingriffselement des Arbeitseinsatzes in Verbindungseingriff mit der mindestens einen ersten Eingriffsstruktur des Verbindungsabschnitts des Schafts. Durch Drehen um die Längsachse des Schafts bzw. des Verbindungsabschnitts und Entnehmen in distaler Richtung wird der Arbeitseinsatz insbesondere nach Art einer Bajonettverbindung von der Verbindungseinrichtung gelöst und von dem Schaft getrennt. Weiter wird ein Arbeitseinsatz gemäß der zweiten Bauart bereitgestellt. Durch Einführen aus axialer Richtung in den Schaft und schließlich Drehen um die Längsachse wird dieser mit dem Schaft verbunden. Der Arbeitseinsatz der zweiten Bauart ist derart unterschiedlich zu dem ersten ausgestaltet, dass das mindestens eine Eingriffselement des zweiten Arbeitseinsatztyps in Verbindungseingriff mit der zweiten Eingriffsstruktur des Verbindungsabschnitts des Schafts gelangt. Dementsprechend ist das mindestens eine Eingriffselement des zweiten Arbeitseinsatztyps unterschiedlich angeordnet und/oder ausgestaltet wie das des ersten Arbeitseinsatztyps. Der Austausch der Arbeitseinsätze kann beispielsweise während eines endoskopischen Eingriffs erfolgen, oder es kann nach einem endoskopischen Eingriff der Arbeitseinsatz der ersten Bauart vom Schaft gelöst werden, eine Reinigung und/oder Sterilisation zumindest des Schafts vorgenommen werden, und vor einem weiteren endoskopischen Eingriff der Arbeitseinsatz der zweiten Bauart mit dem Schaft verbunden werden. Das erfindungsgemäße Verfahren kann weitere Schritte umfassen, etwa die Abnahme und/oder Befestigung eines Handgriffs.

Die voranstehenden Ausführungen bezüglich des Schafts gelten in gleicher Weise auch für das endoskopische Instrument sowie für das endoskopische System und für das Verfahren zum Konfigurieren des endoskopischen Instruments.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen, jeweils schematisch:
Fig. 1 einen Schaft gemäß einer Ausführungsform der vorliegenden Erfindung mit einem in diesen eingesetzten Arbeitseinsatz in einer Seitenansicht;
Fig. 2 eine perspektivische Ansicht des distalen Endabschnitts des Schafts gemäß Fig. 1;
Fig. 3 eine perspektivische Ansicht des Schafts gemäß Fig. 2 mit teilweise eingeführtem Arbeitseinsatz einer ersten Bauart;
Fig. 4 eine perspektivische Ansicht des Schafts gemäß Fig. 2 mit dem vollständig eingeführten und verbundenen Arbeitseinsatz der ersten Bauart;
Fig. 5 eine perspektivische Ansicht des Schafts gemäß Fig. 2 mit teilweise eingeführtem Arbeitseinsatz einer zweiten Bauart;
Fig. 6 eine perspektivische Ansicht des Schafts gemäß Fig. 5 mit vollständig eingeführtem und verbundenen Arbeitseinsatz der zweiten Bauart.

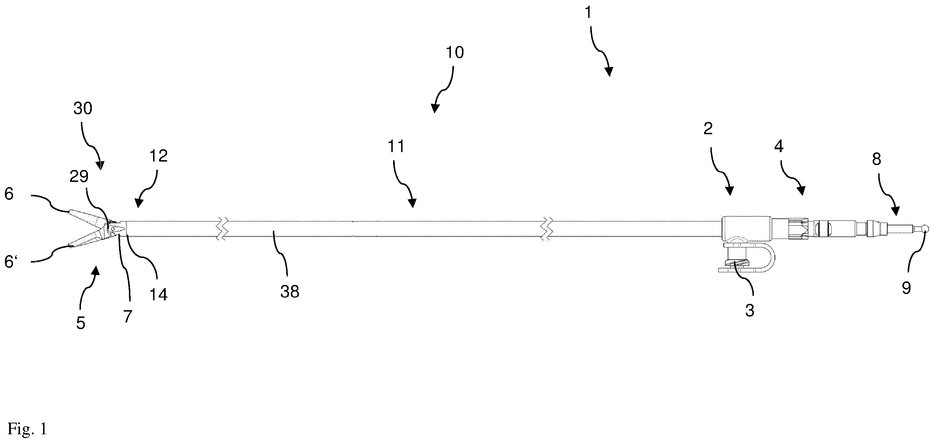

Wie in Fig. 1 dargestellt ist, umfasst ein Schaft 10 für ein medizinisches endoskopisches Instrument 1 gemäß einem Ausführungsbeispiel der vorliegenden Erfindung einen proximalen Abschnitt 2, einen langerstreckten mittleren Abschnitt 11 und einen distalen Endabschnitt 12. Der Schaft 10 ist zur Einführung in einen körperinneren Hohlraum bemessen, beispielsweise zur Einführung in einen Arbeitskanal eines Endoskops, das bei einem endoskopischen Eingriff in den körperinneren Hohlraum eingeführt wird. Der proximale Abschnitt 2 des Schafts 10 kann einen Anschluss 3 aufweisen, beispielsweise einen Spülanschluss, sowie einen in Fig. 1 lediglich beispielhaft dargestellten Verbindungsmechanismus 4 zur Verbindung mit einem nicht dargestellten Handgriff. Der Verbindungsmechanismus 4 kann derart ausgebildet sein, dass der Handgriff drehbar angesetzt werden kann, so dass der Schaft 10 relativ zum Handgriff um die Längsachse des Schafts 10 gedreht werden kann.

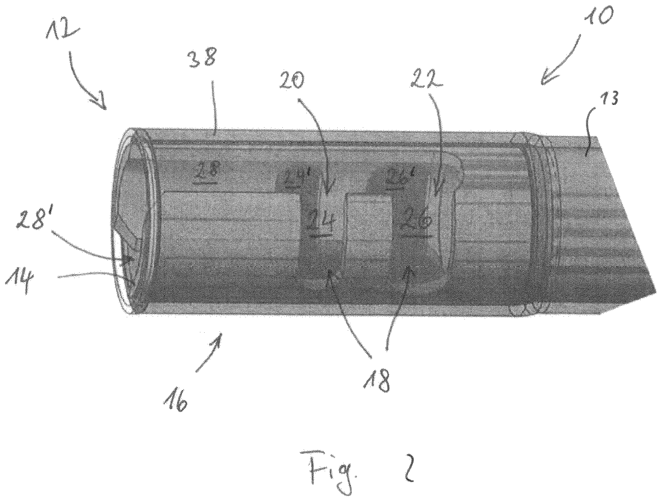

Mit dem distalen Endabschnitt 12 des Schafts 10 ist ein Werkzeug 5 verbunden, das im dargestellten Ausführungsbeispiel als Schere mit zwei relativ zum distalen Endabschnitt 12 des Schafts 10 schwenkbaren Scherenblättern 6, 6' ausgebildet ist. Das Werkzeug 5 ist Teil eines Arbeitseinsatzes 30, der distalseitig in den Schaft 10 eingesetzt ist. Der Arbeitseinsatz 30 umfasst weiterhin eine Basis 7, die distalseitig als Gabel 29 ausgebildet ist, worin die Scherenblätter 6, 6' schwenkbar gelagert sind. Der Arbeitseinsatz 30 liegt mit einem Anschlag der Basis 7 gegen das distale Ende 14 des Schafts 10 an. Wie mit Bezug auf die Figuren 2 bis 6 unten näher erläutert wird, ist die Basis 7 mit einem Einführabschnitt 32 in den distalen Endabschnitt 12 des Schafts 10 eingesetzt und mit diesem verbunden, welcher distale Endabschnitt 12 hierfür als Verbindungsabschnitt 16 ausgebildet ist (s. Fig. 2 bis 6).

Innerhalb des Schafts 10 ist eine Zugstange 8 in Längsrichtung des Schafts 10 verschiebbar angeordnet. Das proximale Ende der Zugstange 8 wird durch ein Verbindungselement gebildet, beispielsweise eine Kugel 9, die mit einem beweglichen Teil des Handgriffs verbunden werden kann, um durch Betätigen des beweglichen Teils die Zugstange 8 in Längsrichtung zu verschieben. Die Zugstange 8 kann sowohl Zug- als auch Schubkräfte in Längsrichtung des Schafts 10 übertragen. Die Zugstange 8 ist gelenkig mit den Scherenblättern 6, 6' verbunden und bildet ebenfalls einen Teil des Arbeitseinsatzes 30. Durch Verschieben der Zugstange 8 in distaler Richtung können die Scherenblätter 6, 6' geöffnet und durch Verschieben in proximaler Richtung geschlossen werden; ebenso ist eine umgekehrte Wirkungsweise der Zugstange 8 denkbar, wobei durch Verschieben der Zugstange 8 in distaler Richtung die Scherenblätter 6, 6' geschlossen und durch Verschieben in proximaler Richtung geöffnet werden können. Der Schaft 10 ist als metallisches Schaftrohr ausgebildet, das von einer elektrisch isolierenden Umhüllung 38 umschlossen sein kann. Am Handgriff können beispielsweise elektrische Anschlüsse für HF-Spannung angeordnet sein.

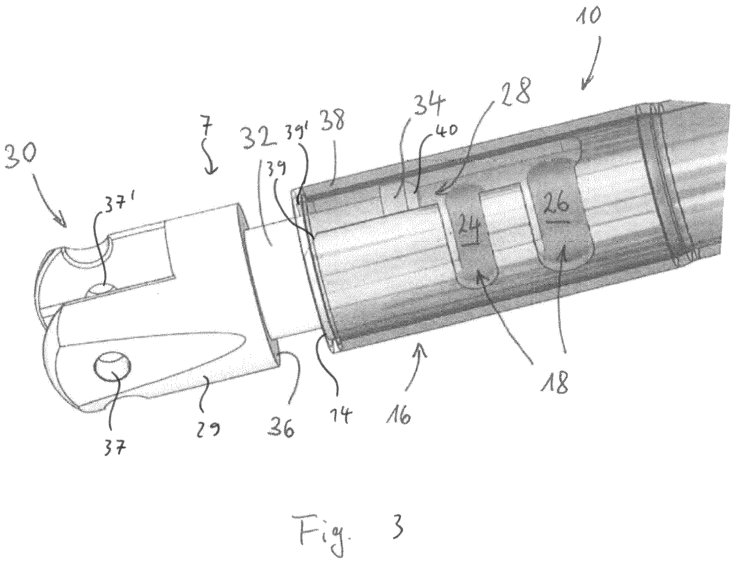

Fig. 2 zeigt eine perspektivische Ansicht des distalen Endabschnitts 12 des Schafts 10, welcher durch ein metallisches Schaftrohr 13 gebildet wird. An dem distalen Endabschnitt 12 ist das distale Ende 14 des Schafts 20 ausgebildet. Der distale Endabschnitt 12 des Schafts 10 bildet einen Verbindungsabschnitt 16. Der Verbindungsabschnitt 16 weist eine Verbindungseinrichtung 18 zur Verbindung eines austauschbaren und in Fig. 2 nicht gezeigten Arbeitseinsatzes mit dem Verbindungsabschnitt 16 auf. Die Verbindungseinrichtung 18 weist hierzu eine Mehrzahl von Eingriffsstrukturen 20, 22 für den Verbindungseingriff unterschiedlicher Bauarten von Arbeitseinsätzen auf. Dabei kann ein Arbeitseinsatztyp, etwa ein Arbeitseinsatz einer ersten Bauart, mit einer ersten Eingriffsstruktur 20 in Verbindungseingriff gebracht werden, und durch Austausch des Arbeitseinsatzes durch einen anderen Arbeitseinsatztyp, etwa einen Arbeitseinsatz einer zweiten Bauart, kann ein Verbindungseingriff mit einer zweiten Eingriffsstruktur 22 erzeugbar sein, was bezugnehmend auf die Figuren 3 bis 6 nachfolgend noch näher erläutert wird.

Die Eingriffsstrukturen 20, 22 sind als Querschlitze 24, 26 ausgebildet. Die Querschlitze 24, 26 verlaufen ausgehend von einem Längsschlitz 28 in einer Umfangsrichtung des Schafts 10. Von dem distalen Ende 14 aus gesehen verlaufen die Querschlitze 24, 26 ausgehend vom Längsschlitz 28 im Uhrzeigersinn. Der Längsschlitz 28 mündet am distalen Ende 14 des Schafts 10. Der Längsschlitz 24 verläuft entlang einer Längsrichtung des Schafts 10 bzw. parallel zu einer Längsachse des Verbindungsabschnitts 16. Eine derartige Verbindungseinrichtung 18 bestehend aus einem Längsschlitz 28 und den Querschlitzen 24, 26 eignet sich zur Herstellung einer Verbindung nach Art eines Bajonettverschlusses.

In Fig. 2 ist weiterhin dargestellt, dass eine Anordnung von einem Längsschlitz 28' und zwei Querschlitzen 24', 26' auch auf einer in Umfangsrichtung um 180° versetzten Position vorgesehen ist. Die Verbindungseinrichtung 18 kann demnach in vorteilhafter Weise zwei bezüglich der Längsachse des Verbindungsabschnitts 16 gegenüberliegend angeordnete Längsschlitze 28, 28' aufweisen, von denen jeweils zwei Querschlitze 24, 26 bzw. 24', 26' ausgehen.

Die Querschlitze 24, 26 sind in einem unterschiedlichen Abstand zum distalen Ende 14 angeordnet und weisen unterschiedliche Breiten auf. Durch die unterschiedlichen Breiten und die unterschiedlichen Abstände zum distalen Ende 14 bilden die Querschlitze 24, 26 eine mechanische Kodierung. Eine solche mechanische Kodierung kann den Verbindungseingriff zwischen einem Arbeitseinsatztyp, der der zweiten Bauart des Arbeitseinsatzes entspricht, und dem ersten Querschlitz 24 blockieren und den Verbindungseingriff zwischen diesem Arbeitseinsatztyp und dem zweiten Querschlitz 26 zulassen. Bei einem anderen Arbeitseinsatztyp, der der ersten Bauart entspricht, kann mit dem ersten Querschlitz 24 ein Verbindungseingriff hergestellt werden, wohingegen zwischen einem solchen Arbeitseinsatztyp und dem zweiten Querschlitz 26 ein Verbindungseingriff blockiert sein kann, was bezugnehmend auf die Figuren 3 bis 6 ebenfalls noch näher erläutert wird. Gleiches gilt für die gegenüberliegend angeordneten Querschlitze 24', 26'.

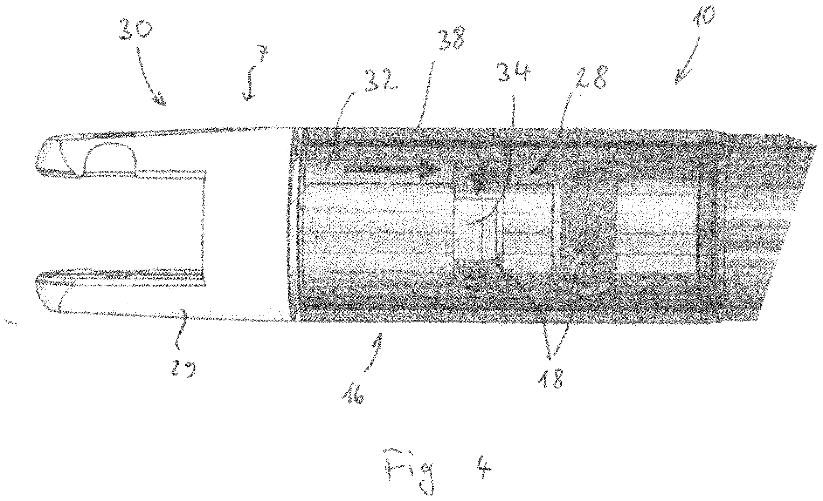

Die Fig. 3 zeigt eine perspektivische Ansicht des Schafts 10 mit teilweise eingeführtem Arbeitseinsatz 30 gemäß der ersten Bauart, und in Fig. 4 ist der Arbeitseinsatz 30 vollständig in den Schaft 10 eingeführt und mit diesem verbunden.

Der Arbeitseinsatz 30 weist eine Basis 7 auf, die eine Gabel 29 und einen näherungsweise zylindrischen Einführabschnitt 32 umfasst. Die Gabel 29 ist zur schwenkbaren Lagerung von hier nicht gezeigten Maulteilen des Werkzeugs 5, etwa der Scherenblätter 6, 6' (s. Fig. 1), ausgebildet und weist hierfür zwei Schwenklager 37, 37' auf. Der Einführabschnitt 32 des Arbeitseinsatzes 30 ist in den Verbindungsabschnitt 16 des Schafts 10 einführbar. An dem Einführabschnitt 32 ist ein Vorsprung 34 oder eine Mehrzahl von Vorsprüngen 34 ausgebildet. Der Vorsprung 34 wird beim Einführen des Einführabschnitts 32 in den Verbindungsabschnitt 16 des Schafts 10 entlang des Längsschlitzes 28 geführt. Im dargestellten Beispiel sind zwei einander gegenüberliegend angeordnete Vorsprünge vorhanden, von denen in Fig. 3 lediglich ein Vorsprung 34 erkennbar ist. Der Vorsprung 34 ist in dem Längsschlitz 28 geführt, und der zweite Vorsprung in dem Längsschlitz 28' (s. Fig. 2).

Nach dem Einführen des Einführabschnitts 32 in den Verbindungsabschnitt 16 des Schafts 10 und geeigneter Führung des Vorsprungs 34 in dem Längsschlitz 28 kann der Vorsprung 34 den ersten Querschlitz 24 erreichen. In dieser Stellung kann der Arbeitseinsatz 30 um die Längsachse des Schafts 10 verdreht werden, so dass der Vorsprung 34 in Verbindungseingriff mit dem ersten Querschlitz 24 gelangt. Zugleich gelangt der durch den gegenüberliegenden Längsschlitz 28' geführte zweite Vorsprung in Verbindungseingriff mit dem gegenüberliegenden Querschlitz 24'. Hierdurch kann der Arbeitseinsatz 30 in Längsrichtung relativ zum Schaft 10 fixiert werden, wie in der Fig. 4 dargestellt ist, wobei in Fig. 4 die axiale und die anschließende in Umfangsrichtung gerichtete Bewegung des Vorsprungs 34 durch Pfeile dargestellt ist. Der Verbindungseingriff zwischen dem Vorsprung 34 und dem Querschlitz 24 kann mit einem hier nicht gezeigten Sicherungsmittel aufrecht erhalten oder nur gegen Überwindung einer vorbestimmten Kraft aufgehoben werden, beispielsweise kann die Zugstange 8, die durch die Basis 7 hindurchgeführt und mit den Maulteilen des Werkzeugs 5 verbunden ist (s. Fig. 1), eine Verdrehsicherung bilden.

Aufgrund der begrenzten Länge des Einführabschnitts 32 sowie der Ausbildung eines kragenförmigen Anschlags 36, an dem der Einführabschnitt 32 endet, kann der Vorsprung 34 des Arbeitseinsatzes 30 lediglich mit dem Querschlitz 24 in Eingriff gebracht werden. Ein weitergehendes Einführen des Einführabschnitts 32 ist durch die Kontaktierung des distalen Endes 14 durch den Anschlag 36 blockiert, so dass der Vorsprung 34 des Arbeitseinsatzes 30 des ersten Arbeitseinsatztyps nicht in Verbindungseingriff mit dem weiter vom distalen Ende 14 beabstandeten zweiten Querschlitz 26 gebracht werden kann.

Der Querschlitz 24 mit der geringeren Breite kann dabei für den Verbindungseingriff mit einem bereits vorhandenen Arbeitseinsatztyp oder mehreren vorhandenen Arbeitseinsatztypen vorgesehen sein, die sich etwa hinsichtlich der Art der Maulteile unterscheiden, bei denen jedoch der Einführabschnitt 32 wie zuvor beschrieben ausgebildet ist. Dementsprechend kann der erste Querschlitz 24 für die Verbindung mit relativ großen Toleranzfeldern ausgebildet sein. Im Verbindungseingriff mit dem Vorsprung 34 des Arbeitseinsatzes 30 kann daher ein verhältnismäßig großes Spiel vorhanden sein. Allerdings wird hierdurch die Rückwärtskompatibilität des Schafts 10 mit bereits vorhandenen Arbeitseinsätzen gewährleistet.

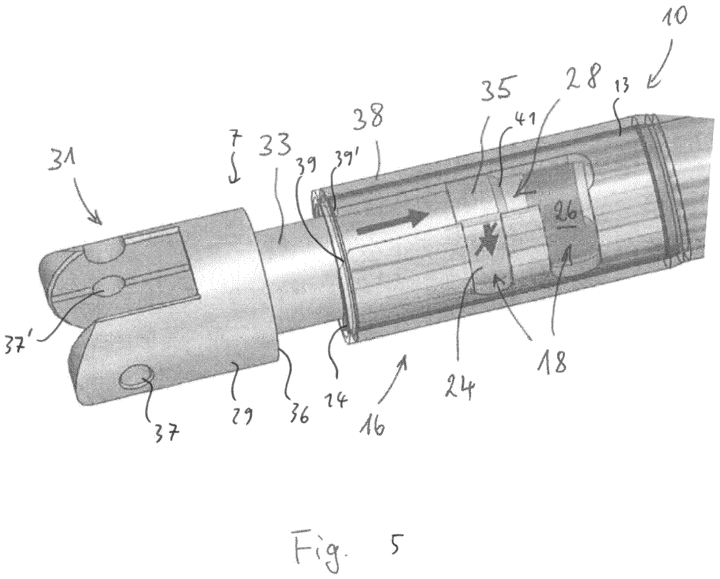

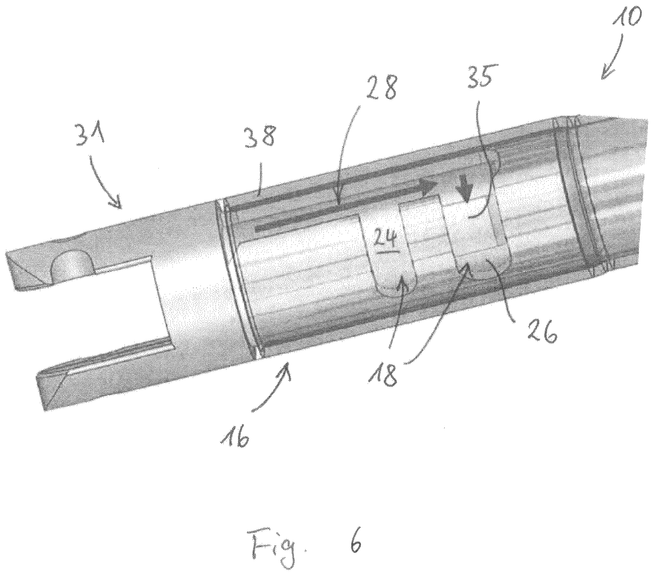

Die Fig. 5 zeigt eine perspektivische Ansicht des Schafts 10 mit einem teilweise eingeführten Arbeitseinsatz 31 einer zweiten Bauart, und in Fig. 6 ist der Arbeitseinsatz 31 vollständig in den Schaft 10 eingeführt und mit diesem verbunden.

Bei dem Arbeitseinsatz 31 handelt es sich um einen Arbeitseinsatz einer zweiten Bauart, die gegenüber dem zuvor beschriebenen Arbeitseinsatz 30 unterschiedlich ausgebildet ist. Der Arbeitseinsatz 31 weist ebenfalls eine Basis 7 auf, die eine Gabel 29 und einen näherungsweise zylindrischen Einführabschnitt 33 umfasst. Die Gabel 29 ist ebenfalls zur schwenkbaren Lagerung von hier nicht gezeigten Maulteilen des Werkzeugs 5, beispielsweise der Scherenblätter 6, 6' oder auch eines andersartigen Werkzeugs wie einer Fasszange, ausgebildet. Der Einführabschnitt 33 des Arbeitseinsatzes 30 ist in den Verbindungsabschnitt 16 des Schafts 10 einführbar. An dem Einführabschnitt 33 ist ein Vorsprung 35 ausgebildet, welcher beim Einführen des Einführabschnitts 33 in den Verbindungsabschnitt 16 des Schafts 10 entlang des Längsschlitzes 28 geführt ist. Im dargestellten Beispiel sind ebenfalls zwei einander gegenüberliegend angeordnete Vorsprünge vorhanden, von denen in Fig. 5 lediglich ein Vorsprung 35 erkennbar ist.

Der Einführabschnitt 33 des Arbeitseinsatzes 31 hat eine größere Länge als der Einführabschnitt 32 des zuvor beschriebenen Arbeitseinsatzes 30 der ersten Bauart. Der Vorsprung 35 ist somit in einem größeren axialen Abstand von dem Anschlag 36 angeordnet als bei dem zuvor beschriebenen ersten Arbeitseinsatztyp. Der Arbeitseinsatz 31 des zweiten Arbeitseinsatztyps unterscheidet sich von dem in den Figuren 2 und 3 gezeigten Arbeitseinsatz 30 des ersten Typs weiterhin betreffend die Dimensionierung des Vorsprungs 35 bzw. der Vorsprünge 35 in der Längsrichtung, wobei der Vorsprung 35 bzw. die Vorsprünge 35 in axialer Richtung größer dimensioniert ist bzw. sind als der Vorsprung 34 bzw. die Vorsprünge 34 des ersten Arbeitseinsatztyps. Auch der Vorsprung 35 wird beim Einführen des Einführabschnitts 33 in den Verbindungsabschnitt 16 des Schafts 10 entlang des jeweiligen Längsschlitzes 28 geführt. Insbesondere kann jeweils ein Vorsprung 35 in einem Längsschlitz 28, 28' (s. Fig. 2) geführt sein.

Nach dem Einführen des Einführabschnitts 33 in den Verbindungsabschnitt 16 des Schafts 10 und geeigneter Führung des Vorsprungs 35 in dem Längsschlitz 28 kann der Vorsprung 35 den Querschlitz 24 erreichen. Aufgrund der größeren Dimensionierung des Vorsprungs 35 in Längsrichtung kann dieser jedoch nicht in den Querschlitz 24, der hierfür eine zu geringe Breite aufweist, hineinbewegt werden. Die Herstellung eines Verbindungseingriffs zwischen dem Vorsprung 35 und dem Querschlitz 24 ist somit blockiert. Die Bewegung des Vorsprungs 35 in axialer Richtung und die blockierte Bewegung in Umfangsrichtung sind in Fig. 5 durch den langen Pfeil bzw. den durchgestrichenen Pfeil angedeutet.

Bei weiterem Einführen des Einführabschnitts 33 in den Verbindungsabschnitt 16 des Schafts 10 und geeigneter Führung des Vorsprungs 35 in dem Längsschlitz 28 kann der Vorsprung 35 den Querschlitz 26 erreichen. In dieser Stellung kann der Arbeitseinsatz 31 um die Längsachse des Schafts 10 verdreht werden, so dass der Vorsprung 35 in Verbindungseingriff mit dem Querschlitz 26 gelangt. Die Bewegung des Vorsprungs 35 in axialer Richtung und anschließend in Umfangsrichtung ist in Fig. 6 durch die Pfeile angedeutet. Hierdurch kann der Arbeitseinsatz 31 in Längsrichtung relativ zum Schaft 10 fixiert werden. Zugleich gelangt der durch den gegenüberliegenden Längsschlitz 28' geführte zweite Vorsprung in Verbindungseingriff mit dem gegenüberliegenden Querschlitz 26'. Der Verbindungseingriff zwischen Vorsprung 34 und Querschlitz 26 kann mit einem hier nicht gezeigten Sicherungsmittel aufrecht erhalten oder nur gegen Überwindung einer vorbestimmten Kraft aufgehoben werden, beispielsweise kann die Zugstange 8, die in entsprechender Weise durch die Basis 7 hindurchgeführt und mit den Maulteilen des Werkzeugs 5 verbunden ist (s. Fig. 1), eine Verdrehsicherung darstellen.

Der Querschlitz 26 mit der größeren Breite und dem größeren Abstand vom distalen Ende 14 kann dabei für den Verbindungseingriff mit neuen Arbeitseinsatztypen vorgesehen sein oder auch mit einem Arbeitseinsatz 31, der ein unterschiedlich zum ersten Arbeitstyp ausgestaltetes Werkzeug aufweist. Dementsprechend kann der Querschlitz 26 für die Verbindung mit relativ engen Toleranzfeldern ausgebildet sein. Im Verbindungseingriff mit dem Vorsprung 35 des Arbeitseinsatzes 31 kann daher ein verhältnismäßig geringes Spiel realisiert werden, so dass ein etwaiger toter Gang verkleinert oder vollständig vermieden werden kann. Wie voranstehend erwähnt, wird jedoch durch den näher zum distalen Ende 14 angeordneten Querschlitz 24 die Rückwärtskompatibilität des Schafts 10 mit vorhandenen Arbeitseinsatztypen gewährleistet.

Zugleich mit dem Eingriff des Vorsprungs 34 bzw. 35 kann der Anschlag 36 des Arbeitseinsatzes 30, 31 jeweils am distalen Ende 14 des Schafts 10 bzw. des Verbindungsabschnitts anliegen. Hierdurch kann die Handhabung beim Verbinden des jeweiligen Arbeitseinsatzes 30, 31 mit dem Schaft 10 mittels der Bajonettverbindung erleichtert werden. Ferner können zur Vereinfachung der Handhabung an der distalen Mündung der Längsschlitze 28, 28' jeweils beidseitige Fasen 39, 39' und an der proximalen Seite der Vorsprünge 34, 35 jeweils eine Abschrägung 40, 41 vorhanden sein (s. Fig. 2 bis 6). Ein Lösen der Bajonettverbindung erfolgt jeweils durch entgegengesetztes Drehen der Basis 7 um die Längsachse und Herausführen des Einführabschnitts 32, 33 in distaler axialer Richtung aus dem Verbindungsabschnitt 16.

Der Schaft 10 kann, einschließlich des Verbindungsabschnitts 16, durch ein starres Schaftrohr gebildet sein, das beispielsweise aus Edelstahl hergestellt ist. Der Schaft 10 im gezeigten Ausführungsbeispiel ferner mit einer Umhüllung 38 ausgestattet, die beispielsweise aus einem Kunststoffmaterial besteht und zur elektrischen Isolierung dienen kann. In den Figuren 2 bis 6 ist die Umhüllung 38 transparent dargestellt. Im gezeigten Ausführungsbeispiel ist die Umhüllung 38 im distalen Endabschnitt 12 des Schafts 10 geringfügig aufgeweitet, um das Einführen des Arbeitseinsatzes 30, 31 und das Schaffen der Bajonettverbindung zu erleichtern.

Es ergibt sich somit insgesamt ein Schaft 10, der eine größere Betriebssicherheit bzw. Betriebsgenauigkeit und zugleich eine vielseitige Verwendbarkeit gewährleistet.

Wie oben anhand von Fig. 1 erläutert worden ist, kann der Schaft 10 zusammen mit einem in diesen verbundenen Arbeitseinsatz 30, 31 einschließlich der Zugstange 8 und ggf. mit einem am proximalen Abschnitt 2 des Schafts 10 angesetzten Handgriff ein medizinisches endoskopisches Instrument 1 bilden. Ferner kann der Schaft 10 zusammen mit mehreren Arbeitseinsätzen 30 und 31 unterschiedlicher Typen ein endoskopisches System, insbesondere ein medizinisches endoskopisches System, bilden, oder Teil eines solchen endoskopischen Systems sein.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Instrument
- 2: Proximaler Abschnitt
- 3: Anschluss
- 4: Verbindungsmechanismus
- 5: Werkzeug
- 6, 6': Scherenblatt
- 7: Basis
- 8: Zugstange
- 9: Kugel
- 10: Schaft
- 11: Mittlerer Abschnitt
- 12: Distaler Endabschnitt
- 13: Schaftrohr
- 14: Distales Ende
- 16: Verbindungsabschnitt
- 18: Verbindungseinrichtung
- 20: Eingriffsstruktur
- 22: Eingriffsstruktur
- 24, 24': Querschlitz
- 26, 26': Querschlitz
- 28, 28': Längsschlitz
- 29: Gabel
- 30: Arbeitseinsatz
- 31: Arbeitseinsatz
- 32: Einführabschnitt
- 33: Einführabschnitt
- 34: Vorsprung
- 35: Vorsprung
- 36: Anschlag
- 37, 37': Schwenklager
- 38: Umhüllung
- 39, 39': Fase
- 40: Abschrägung
- 41: Abschrägung

## Patentansprüche

1. Schaft für ein endoskopisches Instrument (1), mit einem in einem distalen Endbereich des Schafts (10) angeordneten Verbindungsabschnitt (16), wobei an dem Verbindungsabschnitt (16) eine Verbindungseinrichtung (18) zur Verbindung, insbesondere nach Art einer Bajonettverbindung, mit einem austauschbaren Arbeitseinsatz (30, 31) ausgebildet ist, wobei die Verbindungseinrichtung (18) eine erste Eingriffsstruktur (20) für einen Verbindungseingriff eines Eingriffselements eines Arbeitseinsatzes (30) einer ersten Bauart aufweist, **dadurch gekennzeichnet, dass** die Verbindungseinrichtung (18) eine zweite Eingriffsstruktur (22) für einen Verbindungseingriff eines Eingriffselements eines Arbeitseinsatzes (31) einer zweiten Bauart aufweist, wobei die erste und die zweite Eingriffsstruktur (20, 22) unterschiedlich angeordnet und/oder ausgebildet sind, wobei die erste und die zweite Eingriffsstruktur (20, 22) derart unterschiedlich angeordnet und/oder ausgebildet sind, dass die erste Eingriffsstruktur (20) den Verbindungseingriff mit dem Eingriffselement des Arbeitseinsatzes (30) der ersten Bauart zulässt und einen Verbindungseingriff mit dem Eingriffselement des Arbeitseinsatzes (31) der zweiten Bauart blockiert, und dass die zweite Eingriffsstruktur (22) den Verbindungseingriff mit dem Eingriffselement des Arbeitseinsatzes (31) der zweiten Bauart zulässt und einen Verbindungseingriff mit dem Eingriffselement des Arbeitseinsatzes (30) der ersten Bauart blockiert.

2. Schaft nach Anspruch 1, **dadurch gekennzeichnet, dass** die erste und die zweite Eingriffsstruktur (20, 22) derart unterschiedlich angeordnet sind, dass die zweite Eingriffsstruktur (22) in einem von der ersten Eingriffsstruktur (20) unterschiedlichen Abstand von einem distalen Ende (14) des Schafts (10) angeordnet ist.

3. Schaft nach einem der vorhergehenden Ansprüche, **dadurch gekennzeichnet, dass** die Verbindungseinrichtung (18) wenigstens einen Längsschlitz (28) oder eine Längsnut aufweist, der bzw. die zur Längsführung eines Eingriffselements des Arbeitseinsatzes (30, 31) der ersten und/oder der zweiten Bauart ausgebildet ist.

4. Schaft nach einem der vorhergehenden Ansprüche, **dadurch gekennzeichnet, dass** die erste und die zweite Eingriffsstruktur (20, 22) jeweils durch einen quer verlaufenden Querschlitz (24, 24', 26, 26') oder eine Quernut gebildet sind, wobei die Querschlitze (24, 24', 26, 26') bzw. Quernuten zur Längsfixierung eines Eingriffselements eines Arbeitseinsatzes (30, 31) gemäß der ersten bzw. der zweiten Bauart ausgebildet sind.

5. Schaft nach einem der vorhergehenden Ansprüche, **dadurch gekennzeichnet, dass** die erste und die zweite Eingriffsstruktur (20, 22) in einer Längsrichtung des Verbindungsabschnitts (16) beabstandet voneinander ausgebildet sind.

6. Schaft nach einem der vorhergehenden Ansprüche, **dadurch gekennzeichnet, dass** die zweite Eingriffsstruktur (22) unterschiedlich zu der ersten Eingriffsstruktur (20) dimensioniert ist.

7. Schaft nach einem der vorhergehenden Ansprüche, **dadurch gekennzeichnet, dass** die erste und die zweite Eingriffsstruktur (20, 22) unterschiedliche Breiten, Tiefen und/oder Querschnittsformen aufweisen.

8. Schaft nach einem der vorhergehenden Ansprüche in Kombination mit Anspruch 5, **dadurch gekennzeichnet, dass** ein Querschlitz (26, 26') bzw. eine Quernut mit größerem Abstand zu einem distalen Ende (14) des Verbindungsabschnitts (16) eine größere Breite aufweist als ein Querschlitz (24, 24') bzw. eine Quernut mit geringerem Abstand zu dem distalen Ende (14).

9. Schaft nach einem der vorhergehenden Ansprüche, **dadurch gekennzeichnet, dass** die zweite Eingriffsstruktur (22) relativ zu einer Längsachse des Schafts (10) mit zu der ersten Eingriffsstruktur (20) unterschiedlichem radialen Abstand angeordnet ist.

10. Schaft nach einem der vorhergehenden Ansprüche, **dadurch gekennzeichnet, dass** die erste und die zweite Eingriffsstruktur (20, 22) entsprechend unterschiedlichen Toleranzfeldern im Verbindungseingriff mit dem Arbeitseinsatz (30, 31) der ersten bzw. zweiten Bauart ausgebildet sind.

11. Schaft nach einem der vorhergehenden Ansprüche, **dadurch gekennzeichnet, dass** die Verbindungseinrichtung (18) mehrere erste Eingriffsstrukturen (20) und mehrere zweite Eingriffsstrukturen (22) aufweist, die in Umfangsrichtung des Verbindungsabschnitts (16) jeweils zueinander versetzt angeordnet sind.

12. Schaft nach einem der vorhergehenden Ansprüche, **dadurch gekennzeichnet, dass** der Verbindungsabschnitt (16) als distaler Endabschnitt (12) eines Außenschaftes ausgebildet ist.

13. Endoskopisches Instrument, insbesondere medizinisches endoskopisches Instrument, mit einem Schaft (10) nach einem der vorhergehenden Ansprüche sowie einem Arbeitseinsatz (30, 31), der durch die Verbindungseinrichtung (18) lösbar mit dem Verbindungsabschnitt (16) des Schafts (10) verbunden ist.

14. Endoskopisches System, insbesondere endoskopisches medizinisches System, mit einem Schaft (10) nach einem der Ansprüche 1 bis 12 und mit mindestens einem Arbeitseinsatz (30) der ersten Bauart und mindestens einem Arbeitseinsatz (31) der zweiten Bauart.

## Claims

1. Shaft for an endoscopic instrument (1), having a connecting portion (16) arranged in a distal end region of the shaft (10), wherein a connecting device (18) for connection, in particular in the manner of a bayonet connection, to an exchangeable working insert (30, 31) is formed at the connecting portion (16), wherein the connecting device (18) has a first engagement structure (20) for a connection engagement of an engagement element of a working insert (30) of a first type, **characterized in that** the connecting device (18) has a second engagement structure (22) for a connection engagement of an engagement element of a working insert (31) of a second type, wherein the first and the second engagement structures (20, 22) are arranged and/or formed differently.

2. Shaft according to Claim 1, **characterized in that** the first and the second engagement structures (20, 22) are arranged and/or formed differently, in such a way that the first engagement structure (20) permits the connection engagement with the engagement element of the working insert (30) of the first type and blocks a connection engagement with the engagement element of the working insert (31) of the second type, and **in that** the second engagement structure (22) permits the connection engagement with the engagement element of the working insert (31) of the second type and blocks a connection engagement with the engagement element of the working insert (30) of the first type.

3. Shaft according to Claim 1 or 2, **characterized in that** the first and the second engagement structures (20, 22) are arranged differently, in such a way that the second engagement structure (22) is arranged at a distance from a distal end (14) of the shaft (10) different than the distance from the first engagement structure (20).

4. Shaft according to one of the preceding claims, **characterized in that** the connecting device (18) has at least one longitudinal slot (28) or a longitudinal groove, which is formed for the longitudinal guiding of an engagement element of the working insert (30, 31) of the first and/or the second type.

5. Shaft according to one of the preceding claims, **characterized in that** the first and the second engagement structures (20, 22) are each formed by a transversely extending transverse slot (24, 24', 26, 26') or a transverse groove, wherein the transverse slots (24, 24', 26, 26') or transverse grooves are formed for the longitudinal fixing of an engagement element of a working insert (30, 31) according to the first or the second type.

6. Shaft according to one of the preceding claims, **characterized in that** the first and the second engagement structures (20, 22) are formed spaced apart from each other in a longitudinal direction of the connecting portion (16).

7. Shaft according to one of the preceding claims, **characterized in that** the second engagement structure (22) is dimensioned differently than the first engagement structure (20).

8. Shaft according to one of the preceding claims, **characterized in that** the first and the second engagement structures (20, 22) have different widths, depths and/or cross-sectional shapes.

9. Shaft according to one of the preceding claims in combination with Claim 5, **characterized in that** a transverse slot (26, 26') or a transverse groove at a greater distance from a distal end (14) of the connecting portion (16) has a greater width than a transverse slot (24, 24') or a transverse groove at a shorter distance from the distal end (14).

10. Shaft according to one of the preceding claims, **characterized in that** the second engagement structure (22) is arranged, relative to a longitudinal axis of the shaft (10), at a radial distance different than that of the first engagement structure (20).

11. Shaft according to one of the preceding claims, **characterized in that** the first and the second engagement structures (20, 22) are formed in accordance with different tolerance fields in the connection engagement with the working insert (31, 32) of the first or second type.

12. Shaft according to one of the preceding claims, **characterized in that** the connecting device (18) has a plurality of first engagement structures (20) and a plurality of second engagement structures (22) which are each arranged offset from one another in the circumferential direction of the connecting portion (16).

13. Shaft according to one of the preceding claims, **characterized in that** the connecting portion (16) is formed as a distal end portion (12) of an outer shaft.

14. Endoscopic instrument, in particular a medical endoscopic instrument, having a shaft (10) according to one of the preceding claims and having a working insert (30, 31) which is releasably connected to the connecting portion (16) of the shaft (10) by the connecting device (18).

15. Endoscopic system, in particular an endoscopic medical system, having a shaft (10) according to one of Claims 1 to 13 and having at least one working insert (30) of the first type and at least one working insert (31) of the second type.

## Revendications

1. Tige destinée à un instrument endoscopique (1), ladite tige comprenant une portion de liaison (16) disposée dans une région d'extrémité distale de la tige (10), un dispositif de liaison (18) étant conçu pour être relié, en particulier à la manière d'une liaison à baïonnette, à un insert de travail échangeable (30, 31) au niveau de la portion de liaison (16), le dispositif de liaison (18) comportant une première structure d'engagement (20) destinée à un engagement de liaison d'un élément d'engagement d'un insert de travail (30) d'un premier type, **caractérisée en ce que** le dispositif de liaison (18) comporte une deuxième structure d'engagement (22) destinée à un engagement de liaison d'un élément d'engagement d'un insert de travail (31) d'un deuxième type, la première et la deuxième structure d'engagement (20, 22) étant disposées et/ou conçues différemment.

2. Tige selon la revendication 1, **caractérisée en ce que** la première et la deuxième structure d'engagement (20, 22) sont disposées et/ou conçues différemment de telle sorte que la première structure d'engagement (20) permette l'engagement de liaison avec l'élément d'engagement de l'insert de travail (30) du premier type et bloque un engagement de liaison avec l'élément d'engagement de l'insert de travail (31) du deuxième type, et de telle sorte que la deuxième structure d'engagement (22) permette l'engagement de liaison avec l'élément d'engagement de l'insert de travail (31) du deuxième type et bloque un engagement de liaison avec l'élément d'engagement de l'insert de travail (30) du premier type.

3. Tige selon la revendication 1 ou 2, **caractérisée en ce que** la première et la deuxième structure d'engagement (20, 22) sont disposées différemment de telle sorte que la deuxième structure d'engagement (22) soit disposée à une distance d'une extrémité distale (14) de la tige (10) qui est différente de la première structure d'engagement (20).

4. Tige selon l'une des revendications précédentes, **caractérisée en ce que** le dispositif de liaison (18) comporte au moins une fente longitudinale (28), ou une gorge longitudinale, qui est conçue pour le guidage longitudinal d'un élément d'engagement de l'insert de travail (30, 31) du premier type et/ou du deuxième type.

5. Tige selon l'une des revendications précédentes, **caractérisée en ce que** la première et la deuxième structure d'engagement (20, 22) sont formées chacune par une fente transversale (24, 24', 26, 26') s'étendant transversalement ou une gorge transversale, les fentes transversales (24, 24', 26, 26') respectivement gorges transversales étant conçues pour la fixation longitudinale d'un élément d'engagement d'un insert de travail (30, 31) selon le premier ou le deuxième type.

6. Tige selon l'une des revendications précédentes, **caractérisée en ce que** la première et la deuxième structure d'engagement (20, 22) sont formées à distance l'une de l'autre dans une direction longitudinale de la portion de liaison (16).

7. Tige selon l'une des revendications précédentes, **caractérisée en ce que** la deuxième structure d'engagement (22) est dimensionnée de manière différente de la première structure d'engagement (20).

8. Tige selon l'une des revendications précédentes, **caractérisée en ce que** la première et la deuxième structure d'engagement (20, 22) ont des largeurs, des profondeurs et/ou des formes en coupe transversale différentes.

9. Tige selon l'une des revendications précédentes en combinaison avec la revendication 5, **caractérisée en ce qu'**une fente transversale (26, 26') respectivement une gorge transversale située à une plus grande distance d'une extrémité distale (14) de la portion de liaison (16) a une plus grande largeur qu'une fente transversale (24, 24') respectivement une gorge transversale située à une plus petite distance de l'extrémité distale (14).

10. Tige selon l'une des revendications précédentes, **caractérisée en ce que** la deuxième structure d'engagement (22) est disposée à une distance radiale d'un axe longitudinal de la tige (10) qui est différente de celle de la première structure d'engagement (20).

11. Tige selon l'une des revendications précédentes, **caractérisée en ce que** la première et la deuxième structure d'engagement (20, 22) sont conçues conformément à des zones de tolérance différentes dans l'engagement de liaison avec l'insert de travail (30, 31) du premier ou du deuxième type.

12. Tige selon l'une des revendications précédentes, **caractérisée en ce que** le dispositif de liaison (18) comporte une pluralité de premières structures d'engagement (20) et une pluralité de deuxièmes structures d'engagement (22) qui sont chacune disposées en étant décalées les unes des autres dans la direction circonférentielle de la portion de liaison (16).

13. Tige selon l'une des revendications précédentes, **caractérisée en ce que** la portion de liaison (16) est conçue comme une portion d'extrémité distale (12) d'une tige extérieure.

14. Instrument endoscopique, en particulier instrument endoscopique médical, comprenant une tige (10) selon l'une des revendications précédentes et un insert de travail (30, 31) qui est relié de manière amovible à la portion de liaison (16) de la tige (10) par le biais du dispositif de liaison (18).

15. Système endoscopique, notamment système endoscopique médical, comprenant une tige (10) selon l'une des revendications 1 à 13 et au moins un insert de travail (30) du premier type et au moins un insert de travail (31) du deuxième type.
